# EUROPEAN PATENT SPECIFICATION

(11) **EP 3 167 960 B1**
(45) Date of publication and mention of the grant of the patent: **18.12.2019**
(21) Application number: 16193071.4
(22) Date of filing: 10.10.2016
(51) Int. Cl.: B01L 1/02, G01N 1/28, G01N 1/38, B25J 21/00, B01F 11/00, B01F 15/00, B08B 15/02, B25J 21/02

(54) **BIOLOGICAL SAFETY CABINET FOR THE PREPARATION OF BIOLOGICAL SAMPLES, ESPECIALLY FAECES, AND USE OF SUCH A CABINET.**
BIOLOGISCHER SICHERHEITSSCHRANK FÜR DIE HERSTELLUNG VON BIOLOGISCHEN PROBEN, INSBESONDERE AUSSCHEIDUNGEN, UND VERWENDUNG DAVON.
ENCEINTE DE SÉCURITÉ BIOLOGIQUE POUR LA PREPARATION D'ECHANTILLONS BIOLOGIQUES, EN PARTICULIER DE MATIÉRE FÉCALE, AINSI QUE SON UTILISATION.

(30) Priority: 13.10.2015 HU 1500475
(43) Date of publication of application: 17.05.2017
(73) Proprietor: Burgin s.r.o., 1768 Sturovo (SK)
(72) Inventor: KURUCZ, János, 2230 Gyömr (HU)
(74) Representative: Kacsuk, Zsófia

(56) References cited:
- EP-A1- 1 944 359
- WO-A1-2011/062549
- US-A1- 2014 196 411

## Description

The subject of the invention relates to a biological safety cabinet for the preparation of biological samples, i.e. faeces, stored in sample holders.

The subject of the invention also relates to a method for the use of such a safety cabinet.

Clostridium difficile (CD) is a spore-forming bacterium the toxin of certain strains of which injure the mucous membrane of the large intestine, triggering inflammation and cell death there and causing subsequent symptoms involving diarrhoea, physical deterioration and, in serious cases, intestinal necrosis and blood poisoning. CD may be found almost anywhere in our environment, even among normal intestinal bacteria. It causes illness if the gut flora dies out and CD colonises and over-multiplies. This more frequently occurs due to the effect of antibiotic treatment. The past decade has seen a drastic increase in the occurrence of CD infections (CDI). Not only is incidence increasing, but the condition is changing with epidemic-like accumulation. CDI is characterised by high mortality indicators, treatment resistance, recurrence rate and treatment costs.

According to current widespread practices CDI caused by antibiotics is treated with a further course of antibiotics. Guidelines for the treatment of CDI recommend three antibiotics (metronidazole - M, vancomycin - V and fidaxomicin - F). Unfortunately, it is worrying that primary treatment failure occurs when these are administered: 22% (M), 14% (V), and 12% (F)! A further problem is that even if the antibiotic treatment were effective, the illness reoccurs in 13% to 57% of patients. It is not surprising that significant efforts are being made for the elaboration of alternative treatment strategies.

Among the new procedures, faecal microbiota transplant (FMT) is starting to become popular due to excellent efficacy and safety. During the procedure, the homogenized faeces of a healthy donor are transplanted into the patient's intestinal system, where the living intestinal bacteria restore the gut flora, by this eliminating CDI. On the basis of independent randomised, prospective studies FMT significantly surpassed antibiotic therapies in the treatment of relapse cases. The latest European guideline now officially recommends FMT for the treatment of multiply recurring CDI. High-risk patients or those suffering from a serious infection frequently die during the primary infection, but even if recovery is achieved, it is only possible at the price of extended hospitalisation, significant costs and the development of hospital epidemics. It has turned out that FMT used in these patients is also more effective that antibiotic treatment.

In spite of its experimentally certified efficacy, the widespread use of FMT has been prevented by the fact that a tried, tested and safe method for the production of faecal homogenate had not yet existed to date. In the case of the solutions according to the state of the art, the faeces to be transplanted are homogenized in devices that are not specifically designed for the purpose, in household liquidisers for example, and then diluted with saline solution to the desired consistency. The homogenate is then filtered through a gauze sheet, and then filled into an infusion bag. It was recognised that the greatest disadvantage of the method presented is that the homogenisation process is not properly isolated from the environment, and due to this the risk of infection is significant. It was recognised that a further disadvantage is that the liquidiser vessel, in order to ensure the required sterility, must be replaced after every homogenisation, which significantly increases the cost of the method. As the required dilution ratio is set manually by the person performing the homogenisation, it occurs that this is performed imprecisely and the optimum consistency is difficult to reproduce. It was also recognised that a risk of infection also occurs when the ready homogenate is filled into a collection bag.

It is an object of the present invention to provide a biological safety cabinet suitable for the preparation of biological samples - faeces samples - stored in a sample holder, and a method relating to the use of such a biological safety cabinet which is free of the disadvantages of the solutions according to the state of the art, especially the reduction of the risk of infection during the entire process of the preparation of the homogenate.

The invention is based on the recognition that the preparation of the homogenate may be performed isolated from the environment, using a single device, and so the risk of infection and the cost of the method can be significantly reduced. As a consequence of this, satisfying the expectations of modern medicine, FMT may become widely accessible to patients.

In accordance with the invention, the task was solved with the biological safety cabinet according to claim 1 and with the method according to claim 10.

Further advantageous embodiments of the invention are defined in the dependent claims.

Further details of the invention will be explained by way of exemplary embodiments with reference to the figures, wherein
Figure 1a is a schematic perspective view of a preferable embodiment of a biological safety cabinet according to the invention depicted without its housing,
Figure 1b is a schematic perspective view of a preferable embodiment of the housing that may be fixed to the biological safety cabinet according to figure 1a,
Figure 1c is a schematic top view of the embodiment of the biological safety cabinet according to the invention shown in figure 1a,
Figure 2 is a schematic side cross-sectional view of a preferable embodiment of the sample holder according to the invention,
Figure 3 is a schematic synoptic flowchart of the method according to the invention,
Figure 4 is a schematic picture of the elements participating in step 108 of the method according to the invention.

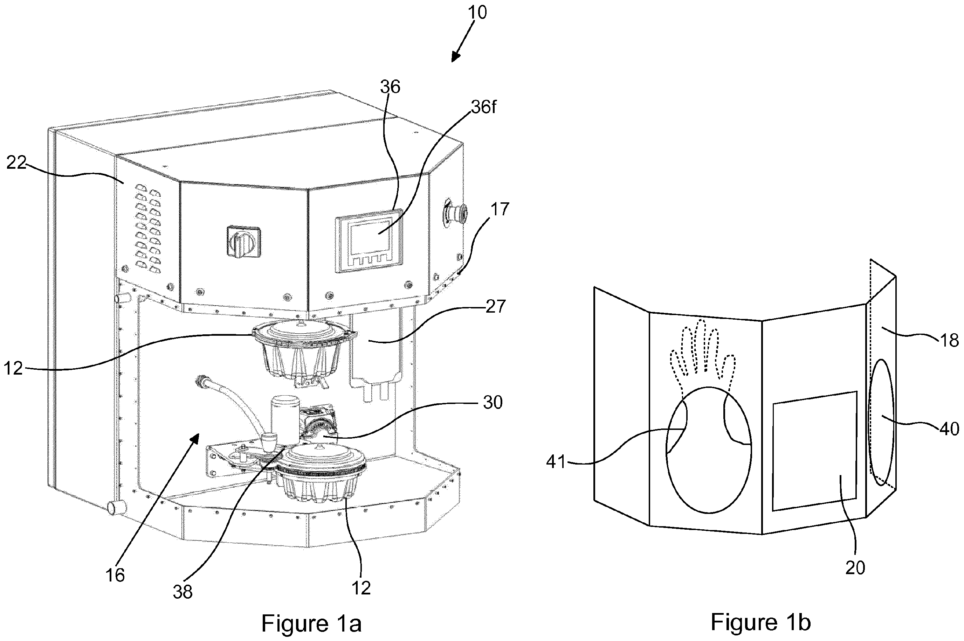

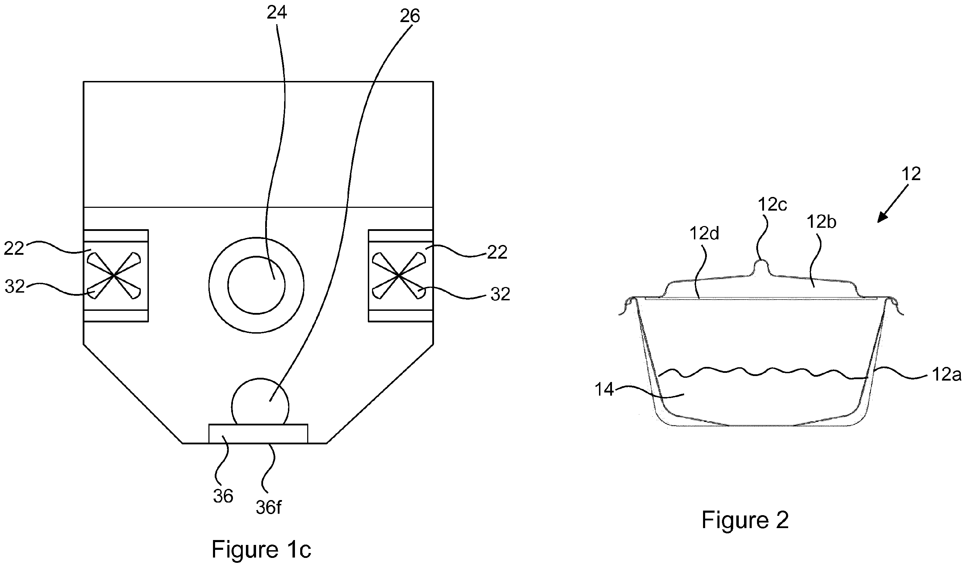

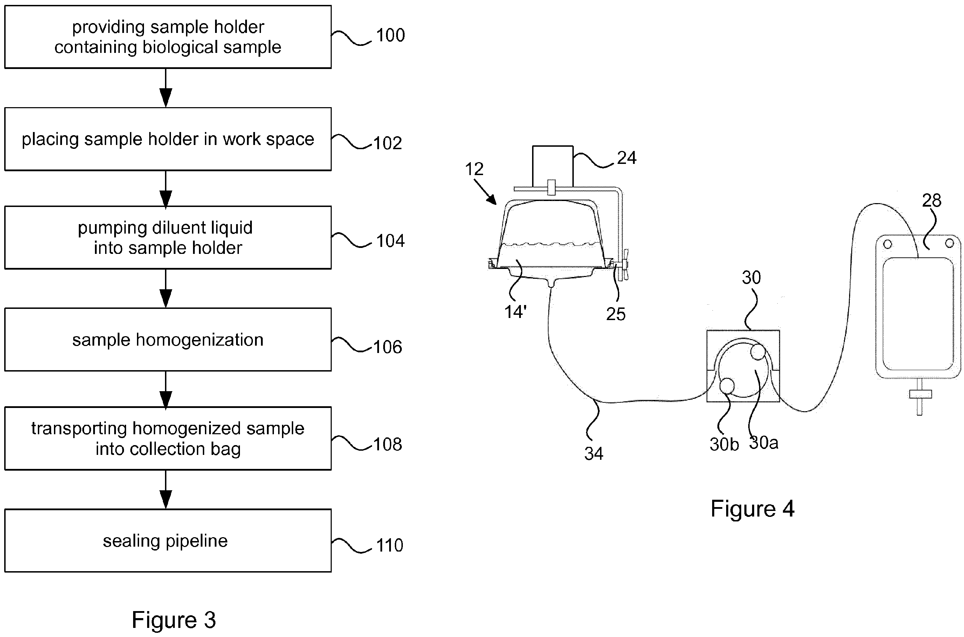

Figures 1a and 1c show schematic perspective and top views of a preferable embodiment of the biological safety cabinet 10 according to the invention, which serves for preparing biological samples 14 (see figure 2), especially faeces, to be stored in a sample holder 12. The biological safety cabinet 10 comprises a housing 18 (see figure 1b) delimiting the protected internal work space 16, an openable and closable insertion door 20 allowing to place the sample holder 12 into the work space 16, a filter unit 22 suitable for cleaning the air leaving the work space 16, a mixing unit 24 serving for homogenising the biological sample 14 stored in the sample holder 12, as well as a peristaltic pump 30 serving for pumping diluent liquid into the sample holder 12 and transporting the prepared diluted and homogenized biological sample 14' from the sample holder 12 to a collection bag 28 (see figure 4). In the case of a preferable embodiment the collection bag 28 is an infusion bag, but, naturally, other storage devices suitable for storing the biological sample 14 may also be conceived. Preferably a high intensity lamp 26, preferably a LED lamp, is arranged in the work space 16 to illuminate the work space 16. A vessel 27 containing the diluent liquid serving for diluting the biological sample 14 is preferably installed in the work space 16 or in a technical space 16' separated from it. The vessel 27 may be an infusion bag, but, naturally, the use of other liquid storage devices is also conceivable. In the case of a preferable embodiment, the biological safety cabinet 10 contains a frame structure 17 that supports the housing 18, which may be made from, for example, metal, plastic, composite or other high-strength material, as is obvious for a person skilled in the art. The housing 18 of the biological safety cabinet 10 is preferably sheet material made from a hermetically sealing substance, such as metal, plastic, glass, etc., which may be fixed to the frame structure 17 with bolts, by welding, soldering, gluing, but naturally, other fixing methods are also conceivable. In the case of an especially preferable embodiment the housing 18 delimiting the work space 16 is transparent or translucent to visible light, which enables the user operating the biological safety cabinet 10 to observe the work space 16. Such a housing 18 may be made from, for example, the aforementioned glass and/or plexi sheet.

An openable and closable insertion door 20 allowing to place the sample holder 12 into the work space 16 is established in the housing 18, which when closed hermetically separates the work space 16 from the space surrounding the biological safety cabinet 10. In the context of the present invention hermetic separation is understood to mean separation that prevents air and pathogens from escaping the work space 16. The insertion door 20 is also preferably made using transparent or translucent plexi and/or glass sheets.

In the case of a preferable embodiment the filter unit 22 contains a HEPA or ULPA filter. HEPA (High-Efficiency Particulate Arrestance) filters are filters that comply with the HEPA standard, which are capable of filtering 99.95% of particles larger than 0.3 µm from the air flowing through them. ULPA (Ultra Low Penetration Air) filters, more effective than HEPA filters, filter out 99.999% of particles larger than 0.1 µm.

In the case of an especially preferable embodiment the biological safety cabinet 10 contains a fan 32 that ensures that the air pressure in the work space 16 is lower than the external air pressure and which removes the air from the work space 16 through the filter unit 22. Optionally, the fan 32 and the filter unit 22 may also be established as a single unit. With respect to its structural arrangement the fan 32 may be radial or axial. As a consequence of the lower air pressure, the air in the work space 16 does not flow outwards even in the case the housing 18 is damaged or if the insertion door 20 is opened, the air only leaves the work space 16 through the filter unit 22.

In the case of a preferable embodiment, the mixing unit 24 is a vibrating mixer serving for mechanically moving the sample holder 12, which contains a supporting axle 25 (see figure 4) enabling the securing and rotating of the sample holder 12.

Such a vibrating mixer may be realised by, for example, an electric vibrator motor, the essence of which is that a weight is fixed to the axle of the electric motor eccentrically with respect to the axle. With the weight rotating around the axle, vibration is created that may be regulated with the speed of rotation, which vibration, in the case of a preferable embodiment, is passed on to the supporting axle 25 in mechanical contact with the vibrator motor and to the sample holder 12 fixed to it. The securing of the sample holder 12 may be realised using, for example, a quick closure, a bolt, a clip or other preferably releasable fixing method, as is obvious for a person skilled in the art. In the case of a preferable embodiment the supporting axle 25 may be rotated in unison with the sample holder 12, the function of which will be elaborated later.

The peristaltic pump 30 is a pump that serves for the continuous or intermittent pumping of substances difficult to transport via a pipeline 34 without any direct contact with the substance, from a suction direction towards a pumping direction. The operation of the exemplary peristaltic pump 30 shown in figure 4 is based on the alternating contracting and relaxing movement of the pipeline 34, as a result of which the content of the pipeline 34 is transported. The peristaltic pump 30 contains a rotor 30a and several rollers 30b fitted onto it, as can be seen in figure 4. As the rotor 30a rotates, the roller 30b compresses the pipeline 34, then as the roller 30b progresses along the pipeline 34, the part of the pipeline 34 left behind regains its original shape and so produces a vacuum. The peristaltic pump 30 is self-priming when dry, in other words even when the pipeline 34 is empty. Before the roller 30b completely leaves the pipeline 34 on the pressure side, another roller 30b compresses it once again on the suction side. During the pumping there is always at least one roller 30 that keeps the pipeline 34 compressed, and prevents the liquid being transported in the pipeline 34 from flowing back from the pressure side to the suction side. In this way the separation of the pressure side from the suction side is ensured even when the peristaltic pump 30 is not in operation. The maximum pressure that may be produced with the peristaltic pump 30 primarily depends on the flexibility of the wall of the pipeline 34. The pipeline 34 may be made from, for example, silicone rubber, Viton, or other flexible material with resistance to fatigue. In the case of a preferable embodiment the speed of rotation of the rotor 30a of the peristaltic pump 30 may be adjusted, due to this the transport direction and/or speed of the peristaltic pump 30 may be regulated. Naturally, a pump different to the peristaltic pump 30 presented here is also suitable for transporting the biological sample 14' into the collection bag 28.

The biological safety cabinet 10 contains a programmable control unit 36 serving for controlling the power supply to the peristaltic pump 30 and the mixing unit 24, and which is preferably electrically connected to them, which may be, for example, a mini computer, dedicated hardware containing a microcontroller, etc. The control unit 36 preferably contains memory for storing programs, as well as a central processing unit (processor) for executing the programs. In the case of an especially preferable embodiment, the control unit 36 contains a user input module 36f, preferably a touch screen, with which the user 50 may input data and select the program for the control unit 36 to execute. The biological safety cabinet 10 according to the invention preferably also contains a scale 38 arranged in the work space 16 serving for accommodating the sample holder 12 and for determining the weight of the sample holder 12. In the case of this embodiment, the scale 38 sends the information relating to the weight of the sample holder 12 to the control unit 36 connected to it with a cable or wireless connection, which processes it and then the stored program uses it as an input parameter, as will be presented in detail later on.

A side cross-section view of a preferable embodiment of the sample holder 12 may be seen in figure 2. The sample holder 12 is preferably a hermetically sealable vessel with ribbed internal walls and/or a ribbed base, which contains a storage part 12a and a lid 12b that may be fixed to the storage part 12a and seals it hermetically. The fixing may be screw fixing, clip fixing or other releasable or non-releasable fixing, as is obvious for a person skilled in the art. The material of the sample holder 12 is preferably plastic, but naturally it may be made from other material of the appropriate strength that allows to safely store the biological sample 14, such as metal or glass. In the case of this embodiment the sample holder 12 contains a connection stub 12c enabling the connection of the pipeline 34, which is sealed before the pipeline 34 is connected. While the pipeline 34 is being connected to the connection stub 12c, the seal on the connection stub 12c is terminated, in such a way, for example, by cutting off the upper part of the connection stub 12c with a special pipe cutting pliers, or, optionally, the connection stub is pierced. The connection of the pipeline 34 to the connection stub 12c is preferably releasable, for example with a screw thread or clip connection. The sample holder 12 contains a filter 12d with a hole size of preferably between 400 to 1000 µm arranged in front of the connection stub 12c and which is suitable for filtering the diluted and homogenized biological sample 14' stored in the sample holder 12. The larger diameter particles remaining in the biological sample 14' after dilution and homogenisation may be filtered out with the help of the filter 12d, as these may cause blockages in the pipeline 34 and have a negative influence on the use of the biological sample 14'.

In the case of a preferable embodiment a manipulation opening 40 is established in the housing 18, and isolator gloves 41 may be fixed to the manipulation opening 40 allowing to manipulate in the work space 16 by hand and hermetically separate the work space 16 and the space part outside of the housing 18, with which the user of the biological safety cabinet 10 may participate in the method according to the invention. The material of the isolator gloves 41 may be, for example, latex, neoprene, nitrile, butyl, Viton, polyurethane, Hypalon, etc., or a combination of these. In the case of a preferable embodiment the isolator gloves 41 are fixed to the manipulation opening 40 in a releasable manner, such as with a hermetically sealing double rubber ring or with another fixing element, as is obvious for a person skilled in the art. Optionally, an embodiment is conceivable in the case of which the biological safety cabinet 10 does not contain isolator gloves 41, instead these are provided and fixed to the manipulation opening 40 by the user herself.

In the following the operation of the biological safety cabinet 10 is presented with reference to figures 3 and 4. During step 100 of the method according to the invention, a sample holder 12 containing a biological sample 14 is provided. In the case of a preferable embodiment, the biological sample 14 to be stored in the sample holder 12 is human and/or animal faeces, which has been placed in the sample holder 12 in advance of the method according to the invention. In the case of a preferable embodiment, while providing the sample holder 12 containing the biological sample 14, the biological sample 14 placed in the sample holder 12 is classified based on its consistency and/or colour and/or composition. After classification, the information relating to the classification is indicated on the sample holder 12, for example, in the form of a printed sticker, appended label or in another way known to a person skilled in the art, then the sample holder 12 is hermetically sealed. It should be noted that optionally the classification described above and the placing of the information relating to the classification may take place independently of the method according to the invention, in advance of step 100, and also the information relating to classification may also contain other elements, such as the weight of the sample holder 12 and/or the biological sample 14, etc.

In the following step 102 the insertion door 20 is opened, the sample holder 12 containing the biological sample 14 is placed in the work space 16, preferably onto the scale 38, then the insertion door 20 is closed. The weight of the sample holder 12 containing the biological sample 14 is measured using the scale 38, then in the knowledge of the empty weight of the sample holder 12, the weight of the biological sample 14 stored in the sample holder 12 is determined.

Using the isolator gloves 41 through the manipulation opening 40 one end of the pipeline 34 threaded through the peristaltic pump 30 is fixed to the connection stub 12c. The other end of the pipeline 34 is connected to the vessel 27 containing the diluent liquid, then in step 104 a specific amount of diluent liquid is transported into the sample holder 12 using the peristaltic pump 30. The connection of the pipeline 34 to the vessel 27 make take place, for example, by fixing a hollow needle to the end of the pipeline 34, with which the rubber plug of the vessel 27 established as an infusion bag is pierced, thereby creating a liquid connection between the pipeline 34 and the vessel 27.

The required amount of the diluent liquid may also be determined by the user by inspecting the biological sample 14, preferably in the knowledge of its weight. In the case of an especially preferable embodiment, the information relating to the weight and/or consistency and/or colour and/or composition of the biological sample 14 is input into the control unit 36. The information input may take place, for example, using the user input module 36f, and/or by the automatic reading of the information indicated on the sample holder 12 (e.g. QR code reading). The required amount of the diluent liquid is automatically determined by the control unit 36 on the basis of the information input in advance, then it is transported into the sample holder 12 by switching on the peristaltic pump 30.

After appropriately diluting the biological sample 14, the pipeline 34 is disconnected from the vessel 27 through the manipulation opening 40 (preferably using the isolator gloves 41), then the sample holder 12 is placed in the mixing unit 24, and the biological sample 14 is homogenized in step 106. In the case of a preferable embodiment the mixing unit 24 is a vibrating mixer containing a vibrator motor. The sample holder 12 is fixed to the supporting axle 25 of the vibrating mixer, as can be seen in figure 4. The speed of rotation and/or the number of rotations and/or the duration of rotation of the vibrator motor are automatically determined by the control unit 36 preferably on the basis of the information relating to the biological sample 14 input in advance during the dilution process. Another possibility is that the user sets the vibration parameters manually, for example, by using the user input module 36f.

The homogenisation of the biological sample 14 is further facilitated by the ribbed structure of the internal wall of the sample holder 12.

Following homogenisation, in step 108 the sample holder is turned around the supporting axle 25, which is preferably arranged horizontally, in such a way that the connection stub 12c faces substantially downwards (see figure 4), then the free end of the pipeline 34 is connected to the collection bag 28 located in the work space 16. Using the peristaltic pump the homogenized biological sample 14' is transported from the sample holder 12 to the collection bag 28. With the connection stub 12c positioned at the bottom, the sample holder 12 is easier to empty, as the hydrostatic pressure of the substantially liquid state homogenized biological sample 14' is added to the pressure exerted by the peristaltic pump 30. In the case of a preferable embodiment, the sample holder 12 contains a filter 12d located in front of the connection stub 12c, and the homogenized sample 14' is transported through this filter 12d into the collection bag 28. The filter 12d filters out the larger particles remaining in the homogenized biological sample 14', by this preventing the pipeline 34 from becoming blocked.

In step 110 following transportation of the homogenized biological sample 14' into the collection bag 28, the pipeline 34 connecting the sample holder 12 and the collection bag 28 in the section between the sample holder 12 and the peristaltic pump 30 and between the collection bag 28 and the peristaltic pump 30 is cut preferably using tube welding, in this way the free ends created with the cut are hermetically sealed at the same time. The cutting and sealing of the pipeline 34 preferably takes place in the work space 16, but, optionally, it may also take place outside of the work space 16. An embodiment is also conceivable in the case of which the pipeline 34 is cut using a separate cutting tool, and the ends created are sealed with sealing elements (such as plugs, tube clamps). Optionally, the homogenized biological sample 14' remaining in the middle part of the pipeline 34 obtained when it is cut and sealed at both ends may be further examined in the laboratory.

The risk of infection may be reduced by sealing the pipeline 34 connected to the sample holder 12. The sample holder 12 is preferable of a single use design, in other words after the homogenized biological sample 14' has been transported into the collection bag 28, the emptied and sealed sample holder 12 may be placed in the appropriate waste collection container. With the sample holder 12 having a single use design, appropriate sterility and more comfortable working conditions may be ensured for the user.

After the connected pipeline 34 has been sealed, the collection bag 28 containing the homogenized biological sample 14' may be transported to the location of use.

Various modifications to the above disclosed embodiments will be apparent to a person skilled in the art without departing from the scope of protection determined by the attached claims.

## Claims

1. Biological safety cabinet (10) for the preparation of faeces (14) stored in sample holders (12), **characterised by** comprising:
- a sample holder (12) for storing faeces (14) therein;
- a housing (18) delimiting a protected internal work space (16),
- an openable and closable insertion door (20) allowing placement of the sample holder (12) into the work space (16), the work space (16) being hermetically separated from a space surrounding the biological safety cabinet (10) when the insertion door (20) is closed,
- a filter unit (22) suitable for cleaning the air leaving the work space (16),
- a mixing unit (24) for homogenising the faeces (14) stored in the sample holder (12),
- a peristaltic pump (30) for pumping diluent liquid (26) into the sample holder (12) and transporting the prepared diluted and homogenized faeces (14') from the sample holder (12) to a collection bag (28), and
- a programmable control unit (36) for controlling the power supply to the peristaltic pump (30) and the mixing unit (24).

2. Biological safety cabinet (10) according to any of claim 1, **characterised by** that the sample holder (12) is a hermetically sealable vessel with ribbed internal walls.

3. Biological safety cabinet (10) according to claim 1 or 2, **characterised by** that the mixing unit (24) is a vibrating mixer for mechanically moving the sample holder (12), which vibrating mixer preferably contains a supporting axle (25) for securing the sample holder (12) and permitting the rotation of the sample holder (12).

4. Biological safety cabinet (10) according to any of claims 1 to 3, **characterised by** that the sample holder (12) is provided for storing human and/or animal faeces.

5. Biological safety cabinet (10) according to any of claims 1 to 4, **characterised by** that the sample holder (12) contains a connection stub (12c) enabling the connection of a pipeline (34) and preferably the sample holder (12) contains a filter (12d) arranged in front of the connection stub (12c) suitable for filtering the faeces (14) stored in it, the hole size of which is preferably between 400 to 1000 µm.

6. Biological safety cabinet (10) according to any of claims 1 to 5, **characterised by** comprising a scale (38), arranged in the work space (16) for the reception of the sample holder (12) and/or a fan (32) providing lower air pressure in the work space (16) than the external air pressure.

7. Biological safety cabinet (10) according to any of claims 1 to 6, **characterised by** that the transport direction and/or the speed of the peristaltic pump (30) is adjustable.

8. Biological safety cabinet (10) according to any of claims 1 to 7, **characterised by** that the control unit (36) has a user input module (36f), preferably in the form of a touch screen.

9. Biological safety cabinet (10) according to any of claims 1 to 8, **characterised by** that a manipulation opening (40) is formed in the housing (18), and isolator gloves (41) are fixed to the manipulation opening (40) allowing to manipulate in the work space (16) by hand and hermetically separate the work space (16) and the space part outside of the housing (18).

10. Method for the use of the biological safety cabinet (10) according to claims 1 to 9, **characterised by**
- providing a sample holder (12) containing faeces (14),
- placing the sample holder (12) containing the faeces (14) into the hermetically separated work space (16) of the biological safety cabinet (10) through the insertion door (20) of the biological safety cabinet (10),
- pumping a determined amount of diluent liquid (26) into the sample holder (12) using the peristaltic pump (30) controlled by the programmable control unit (36),
- placing the sample holder (12) in the mixing unit (24) and homogenizing the faeces (14) with the mixing unit (24) controlled by the programmable control unit (36), and
- transporting the homogenized faeces (14') from the sample holder (12) to the collection bag (28) using the peristaltic pump (30).

11. Method according to claim 10, **characterised by** measuring the weight of the sample holder (12) containing the faeces (14), then determining the weight of the faeces (14), and
automatically determining the required amount of the diluent liquid (26) by the control unit 36 on the basis of the weight of the faeces (14) and on the basis of the consistency and/or colour and/or composition of the faeces (14).

12. Method according to claim 10 or 11, **characterised by** that the following steps are performed while providing the sample holder (12) containing the faeces (14):
qualifying the faeces (14) placed in the sample holder (12) based on its consistency and/or colour and/or composition,
indicating the information relating to the classification on the sample holder (12),
sealing the sample holder (12) hermetically.

13. Method according to any of claims 10 to 12, **characterised by** that the mixing unit (24) is a vibrating mixer containing a vibrator motor, in the case of which the speed of rotation and/or the number of rotations and/or the duration of rotation is controlled by the pre-programmed control unit (36).

14. Method according to any of claims 10 to 13, **characterised by** that the sample holder (12) contains a filter (12d), and the homogenized faeces (14') is transported through the filter (12d), via a pipeline (34) providing liquid connection between the sample holder (12) and the collection bag (28), into the collection bag (28).

15. Method according to any of claims 10 to 14, **characterised by** that after the homogenized faeces (14') has been transported into the collection bag (28) cutting the pipeline (34) connecting the sample holder (12) with the collection bag (28) at a region between the sample holder (12) and the peristaltic pump (30) and at a region between the collection bag (28) and the peristaltic pump (30), and hermetically sealing the free ends created by the cutting.

## Patentansprüche

1. Biologische Sicherheitswerkbank (10) zur Vorbereitung von Fäkalien (14), die in Probenhaltern (12) aufbewahrt werden, **dadurch gekennzeichnet, dass** sie Folgendes umfasst:
- einen Probenhalter (12) zur Aufbewahrung von Fäkalien (14) darin;
- ein Gehäuse (18), das einen geschützten internen Arbeitsraum (16) begrenzt,
- eine öffenbare und verschließbare Einführtür (20), die das Platzieren des Probenhalters (12) in den Arbeitsraum (16) ermöglicht, wobei der Arbeitsraum (16) hermetisch von einem Raum um die biologische Sicherheitswerkbank (10) herum getrennt ist, wenn die Einführtür (20) geschlossen ist,
- eine Filtereinheit (22), die zum Reinigen der den Arbeitsraum (16) verlassenden Luft geeignet ist,
- eine Mischeinheit (24) zum Homogenisieren der im Probenhalter (12) aufbewahrten Fäkalien (14),
- eine Peristaltikpumpe (30) zum Pumpen von Verdünnungsflüssigkeit (26) in den Probenhalter (12) und zum Transportieren der vorbereiteten verdünnten und homogenisierten Fäkalien (14') vom Probenhalter (12) zu einem Sammelbeutel (28), und
- eine programmierbare Steuereinheit (36) zum Steuern der Stromzufuhr zur Peristaltikpumpe (30) und zur Mischeinheit (24).

2. Biologische Sicherheitswerkbank (10) nach einem der Ansprüche 1, **dadurch gekennzeichnet, dass** der Probenhalter (12) ein hermetisch verschließbares Gefäß mit gerippten Innenwänden ist.

3. Biologische Sicherheitswerkbank (10) nach Anspruch 1 oder 2, **dadurch gekennzeichnet, dass** die Mischeinheit (24) ein Vibrationsmischer zum mechanischen Bewegen des Probenhalters (12) ist, der vorzugsweise eine Stützachse (25) zur Befestigung des Probenhalters (12) und zur Drehung des Probenhalters (12) enthält.

4. Biologische Sicherheitswerkbank (10) nach einem der Ansprüche 1 bis 3, **dadurch gekennzeichnet, dass** der Probenhalter (12) für die Aufbewahrung von menschlichen und/oder tierischen Fäkalien vorgesehen ist.

5. Biologische Sicherheitswerkbank (10) nach einem der Ansprüche 1 bis 4, **dadurch gekennzeichnet, dass** der Probenhalter (12) einen Anschlussstutzen (12c) enthält, der den Anschluss einer Rohrleitung (34) ermöglicht, und vorzugsweise der Probenhalter (12) einen vor dem Anschlussstutzen (12c) angeordneten Filter (12d) enthält, der zum Filtern der darin aufbewahrten Fäkalien (14) geeignet ist, dessen Lochgröße vorzugsweise zwischen 400 und 1000 µm liegt.

6. Biologische Sicherheitswerkbank (10) nach einem der Ansprüche 1 bis 5, **dadurch gekennzeichnet, dass** sie eine im Arbeitsraum (16) angeordnete Waage (38) zur Aufnahme des Probenhalters (12) und/oder einen Ventilator (32) umfasst, der einen niedrigeren Luftdruck im Arbeitsraum (16) als den Außenluftdruck bereitstellt.

7. Biologische Sicherheitswerkbank (10) nach einem der Ansprüche 1 bis 6, **dadurch gekennzeichnet, dass** die Förderrichtung und/oder die Drehzahl der Peristaltikpumpe (30) einstellbar ist.

8. Biologische Sicherheitswerkbank (10) nach einem der Ansprüche 1 bis 7, **dadurch gekennzeichnet, dass** die Steuereinheit (36) ein Benutzereingabemodul (36f) aufweist, vorzugsweise in Form eines Touchscreens.

9. Biologische Sicherheitswerkbank (10) nach einem der Ansprüche 1 bis 8, **dadurch gekennzeichnet, dass** im Gehäuse (18) eine Manipulationsöffnung (40) ausgebildet ist und an der Manipulationsöffnung (40) Isolatorhandschuhe (41) befestigt sind, die eine manuelle Manipulation im Arbeitsraum (16) ermöglichen und den Arbeitsraum (16) und den Raumteil außerhalb des Gehäuses (18) hermetisch trennen.

10. Verfahren zur Nutzung der biologischen Sicherheitswerkbank (10) nach den Ansprüchen 1 bis 9, **gekennzeichnet durch**
- Bereitstellen eines Probenhalters (12), der Fäkalien (14) enthält,
- Platzieren des Probenhalters (12) mit den Fäkalien (14) in den hermetisch getrennten Arbeitsraum (16) der biologischen Sicherheitswerkbank (10) durch die Einführtür (20) der biologischen Sicherheitswerkbank (10),
- Pumpen einer bestimmten Menge an Verdünnungsflüssigkeit (26) in den Probenhalter (12) unter Verwendung der von der programmierbaren Steuereinheit (36) gesteuerten Peristaltikpumpe (30),
- Platzieren des Probenhalters (12) in der Mischeinheit (24) und Homogenisieren der Fäkalien (14) mit der von der programmierbaren Steuereinheit (36) gesteuerten Mischeinheit (24), und
- Transportieren der homogenisierten Fäkalien (14') aus dem Probenhalter (12) in den Sammelbeutel (28) unter Verwendung der Peristaltikpumpe (30).

11. Verfahren nach Anspruch 10, **gekennzeichnet durch** das Messen des Gewichts des Probenhalters (12), der die Fäkalien (14) enthält, und das anschließende Bestimmen des Gewichts der Fäkalien (14), und
das automatische Bestimmen der erforderlichen Menge der Verdünnungsflüssigkeit (26) durch die Steuereinheit 36 auf der Grundlage des Gewichts der Fäkalien (14) und auf der Grundlage der Konsistenz und/oder Farbe und/oder Zusammensetzung der Fäkalien (14).

12. Verfahren nach Anspruch 10 oder 11, **dadurch gekennzeichnet, dass** die folgenden Schritte durchgeführt werden, während der Probenhalter (12) mit den Fäkalien (14) bereitgestellt wird:
Qualifizieren der in den Probenhalter (12) platzierten Fäkalien (14) aufgrund ihrer Konsistenz und/oder Farbe und/oder Zusammensetzung,
Angeben der Informationen über die Klassifizierung auf dem Probenhalter (12),
hermetisches Verschließen des Probenhalters (12).

13. Verfahren nach einem der Ansprüche 10 bis 12, **dadurch gekennzeichnet, dass** die Mischeinheit (24) ein Vibrationsmischer ist, der einen Vibrationsmotor enthält, bei dem die Drehzahl und/oder die Anzahl der Umdrehungen und/oder die Dauer der Drehung durch die vorprogrammierte Steuereinheit (36) gesteuert wird.

14. Verfahren nach einem der Ansprüche 10 bis 13, **dadurch gekennzeichnet, dass** der Probenhalter (12) einen Filter (12d) enthält und die homogenisierten Fäkalien (14') durch den Filter (12d) über eine Rohrleitung (34), die eine Flüssigkeitsverbindung zwischen dem Probenhalter (12) und dem Sammelbeutel (28) herstellt, in den Sammelbeutel (28) transportiert wird.

15. Verfahren nach einem der Ansprüche 10 bis 14, **dadurch gekennzeichnet, dass** nach dem Transport der homogenisierten Fäkalien (14') in den Sammelbeutel (28) die Rohrleitung (34), die den Probenhalter (12) mit dem Sammelbeutel (28) verbindet, in einem Bereich zwischen dem Probenhalter (12) und der Peristaltikpumpe (30) und in einem Bereich zwischen dem Sammelbeutel (28) und der Peristaltikpumpe (30) durchgeschnitten wird, und die durch den Schnitt erzeugten freien Enden hermetisch verschlossen werden.

## Revendications

1. Enceinte de sécurité biologique (10) pour la préparation de fèces (14) stockées dans des porte-échantillons (12), **caractérisée par le fait qu'**elle comprend :
- un porte-échantillons (12) pour stocker les fèces (14) à l'intérieur ;
- un boîtier (18) délimitant un espace de travail interne protégé (16) ;
- une porte d'insertion ouvrable et fermable (20) permettant de placer le porte-échantillons (12) dans l'espace de travail (16), l'espace de travail (16) étant séparé hermétiquement d'un espace entourant l'enceinte de sécurité biologique (10) quand la porte d'insertion (20) est fermée,
- une unité de filtre (22) adaptée pour purifier l'air quittant l'espace de travail (16),
- une unité de mélange (24) pour homogénéiser les fèces (14) stockées dans le porte-échantillons (12),
- une pompe péristaltique (30) pour pomper le liquide diluant (26) dans le porte-échantillons (12) et transporter les fèces diluées et homogénéisées préparées (14') du porte-échantillons (12) à un sac de collecte (28), et
- une unité de commande programmable (36) pour commander l'alimentation en énergie vers la pompe péristaltique (30) et l'unité de mélange (24).

2. Enceinte de sécurité biologique (10) selon la revendication 1, **caractérisée par le fait que** le porte-échantillons (12) est un récipient hermétiquement scellable avec des parois internes nervurées.

3. Enceinte de sécurité biologique (10) selon l'une des revendications 1 ou 2, **caractérisée par le fait que** l'unité de mélange (24) est un mélangeur à vibration pour déplacer mécaniquement le porte-échantillons (12), lequel mélangeur à vibration contient de préférence un essieu porteur (25) pour fixer le porte-échantillons (12) et permettre la rotation du porte-échantillons (12).

4. Enceinte de sécurité biologique (10) selon l'une quelconque des revendications 1 à 3, **caractérisée par le fait que** le porte-échantillons (12) est prévu pour stocker des fèces humaines et/ou animales.

5. Enceinte de sécurité biologique (10) selon l'une quelconque des revendications 1 à 4, **caractérisée par le fait que** le porte-échantillons (12) contient une embase de raccordement (12c) permettant la connexion d'une conduite (34) et de préférence le porte-échantillons (12) contient un filtre (12d) disposé devant l'embase de raccordement (12c) adapté pour filtrer les fèces (14) stockées à l'intérieur, dont la taille du trou est de préférence entre 400 et 1000 µm.

6. Enceinte de sécurité biologique (10) selon l'une quelconque des revendications 1 à 5, **caractérisée par le fait qu'**elle comprend une balance (38), disposée dans l'espace de travail (16) pour la réception du porte-échantillons et/ou un ventilateur (32) fournissant une pression d'air inférieure à la pression d'air externe dans l'espace de travail (16).

7. Enceinte de sécurité biologique (10) selon l'une quelconque des revendications 1 à 6, **caractérisée par le fait que** la direction de transport et/ou la vitesse de la pompe péristaltique est ajustable.

8. Enceinte de sécurité biologique (10) selon l'une quelconque des revendications 1 à 7, **caractérisée par le fait que** l'unité de commande (36) a un module de saisie utilisateur (36f), de préférence sous la forme d'un écran tactile.

9. Enceinte de sécurité biologique (10) selon l'une quelconque des revendications 1 à 8, **caractérisée par le fait qu'**une ouverture de manipulation (40) est formée dans le boîtier (18), et des gants isolants (41) sont fixés à l'ouverture de manipulation (40) permettant de manipuler manuellement dans l'espace de travail (16) et de séparer hermétiquement l'espace de travail (16) et la partie de l'espace à l'extérieur du boîtier (18).

10. Procédé d'utilisation de l'enceinte de sécurité biologique (10) selon les revendications 1 à 9, **caractérisé par** :
- la fourniture d'un porte-échantillons (12) contenant les fèces (14),
- le placement du porte-échantillons (12) contenant les fèces (14) dans l'espace de travail (16) séparé hermétiquement de l'enceinte de sécurité biologique (10) par l'intermédiaire de la porte d'insertion (20) de l'enceinte de sécurité biologique (10),
- le pompage d'une quantité déterminée de liquide diluant (26) vers le porte-échantillons (12) à l'aide de la pompe péristaltique (30) commandée par l'unité de commande programmable (36),
- le placement du porte-échantillons (12) dans l'unité de mélange (24) et l'homogénéisation des fèces (14) avec l'unité de mélange (24) commandée par l'unité de commande programmable(36), et
- le transport des fèces homogénéisées (14') du porte-échantillons à la poche de collecte (28) à l'aide de la pompe péristaltique (30).

11. Procédé selon la revendication 10, **caractérisé par** la mesure du poids du porte-échantillons (12) contenant les fèces (14), puis la détermination du poids des fèces (14), et
la détermination automatique de la quantité de liquide diluant (26) requise par l'unité de commande (36) sur la base du poids des fèces (14) et sur la base de la consistance et/ou de la couleur et/ou de la composition des fèces (14).

12. Procédé selon l'une des revendications 10 ou 11, **caractérisé par le fait que** les étapes suivantes sont réalisées tout en fournissant le porte-échantillons (12) contenant les fèces (14) :
qualifier les fèces (14) placées dans le porte-échantillons (12) sur la base de leur consistance et/ou de leur couleur et/ou de leur composition,
indiquer l'information relative à la classification sur le porte-échantillons (12),
sceller le porte-échantillons (12) hermétiquement.

13. Procédé selon l'une quelconque des revendications 10 à 12, **caractérisé par le fait que** l'unité de mélange (24) est un mélangeur à vibration contenant un moteur à vibration, dans le cas duquel la vitesse de rotation et/ou le nombre de rotations et/ou la durée de rotation est commandée par l'unité de commande préprogrammée (36).

14. Procédé selon l'une quelconque des revendications 10 à 13, **caractérisé par le fait que** le porte-échantillons (12) contient un filtre (12d), et les fèces homogénéisées (14') sont transportées à travers le filtre (12d), par l'intermédiaire d'une conduite (34) fournissant une connexion liquide entre le porte-échantillons (12) et la poche de collecte (28), dans la poche de collecte (28).

15. Procédé selon l'une quelconque des revendications 10 à 14, **caractérisé par le fait qu'**après que les fèces homogénéisées (14') ont été transportées dans la poche de collecte (28), couper la conduite (34) connectant le porte-échantillons (12) avec la poche de collecte (28) au niveau d'une région entre le porte-échantillons (12) et la pompe péristaltique (30) et au niveau d'une région entre la poche de collecte (28) et la pompe péristaltique (30), et sceller hermétiquement les extrémités libres créées par la découpe.
